(19) Europäisches Patentamt
European Patent Office
Office européen des brevets

(11) EP 1 430 868 B1

(12) # FASCICULE DE BREVET EUROPEEN

(45) Date de publication et mention de la délivrance du brevet:
**08.03.2006 Bulletin 2006/10**

(51) Int Cl.:
***A61K 8/81*** *(2006.01)* ***A61K 8/87*** *(2006.01)*
***A61K 8/90*** *(2006.01)* ***A61K 8/92*** *(2006.01)*
***A61Q 1/10*** *(2006.01)*

(21) Numéro de dépôt: **03292939.0**

(22) Date de dépôt: **26.11.2003**

(54) **Composition de revêtement des fibres kératiniques présentant un caractère filant**

Dickflüssige Beschichtungszusammensetzung für keratinische Fasern

Coating composition for keratinic fibers with a ropy character

(84) Etats contractants désignés:
**AT BE BG CH CY CZ DE DK EE ES FI FR GB GR HU IE IT LI LU MC NL PT RO SE SI SK TR**

(30) Priorité: **20.12.2002 FR 0216384**

(43) Date de publication de la demande:
**23.06.2004 Bulletin 2004/26**

(73) Titulaire: **L'OREAL**
**75008 Paris (FR)**

(72) Inventeurs:
- **Jager Lezer, Nathalie**
**91370 Verrieres-Le-Buisson (FR)**
- **De la Poterie, Valérie**
**77820 Le Châtelet-en-Brie (FR)**

(74) Mandataire: **Boulard, Denis**
**L'OREAL - D.I.P.I.**
**25-29 Quai Aulagnier**
**92600 Asnières (FR)**

(56) Documents cités:

| | |
|---|---|
| **EP-A- 0 637 600** | **EP-A- 0 638 307** |
| **EP-A- 0 953 332** | **EP-A- 1 258 238** |
| **EP-A- 1 277 463** | **EP-A- 1 281 384** |
| **EP-A- 1 281 385** | **EP-A- 1 396 256** |
| **EP-A- 1 417 951** | **US-A- 4 071 615** |
| **US-A- 5 601 808** | |

Remarques:
Le dossier contient des informations techniques présentées postérieurement au dépôt de la demande et ne figurant pas dans le présent fascicule.

EP 1 430 868 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet une composition cosmétique comprenant un agent épaississant, ladite composition présentant un caractère filant spécifique. Cette composition est destinée au revêtement des fibres kératiniques telles que les cils, les sourcils et les cheveux d'êtres humains ou bien encore aux faux-cils.
La composition est en particulier un produit de maquillage des fibres kératiniques, une base de maquillage des fibres kératiniques, notamment des cils, encore appelé base coat, un produit à appliquer sur un maquillage, dit encore top coat, ou un produit de traitement cosmétique des fibres kératiniques, ou bien encore un produit pour les sourcils. De façon plus spécifique, la composition est un produit de maquillage des cils appelé mascara.

Les compositions de revêtement des cils, appelées mascaras, comprennent généralement un extrait sec qui est en majeure partie une phase grasse dispersée constituée d'une ou plusieurs cires afin d'apporter de la matière sur les cils, ainsi qu'un gainage des cils, et donc un résultat maquillage plus ou moins volumateur (le maquillage des cils est plus ou moins épais).
Néanmoins, l'augmentation de l'extrait sec dans une composition entraîne une augmentation de la consistance de la composition et une mauvaise dispersion des particules solides. De ce fait, le film de maquillage présente un aspect granuleux, non homogène et forme des paquets sur les cils. Le résultat maquillage obtenu au final est inesthétique.
De plus, ces compositions ne permettent pas d'obtenir un effet d'allongement satisfaisant du cil ; on connaît par exemple du document EP 1281384 des mascaras comprenant une teneur élevée en cire mais qui ne confèrent pas aux cils un effet allongeant suffisant.

Il existe donc un besoin pour une composition de revêtement des fibres kératiniques, notamment des cils, qui permette d'obtenir un bon allongement des cils, et qui dépose sur les fibres kératiniques un film de composition lisse, homogène.

Le but de la présente invention est, entre autre, de satisfaire aux exigences mentionnés plus haut en proposant une composition de maquillage des fibres kératiniques, notamment des cils, conduisant à un film de maquillage lisse et homogène et permettant l'obtention d'un effet allongeant des cils.

Les inventeurs ont découvert de manière surprenante que ce but est atteint grâce à une composition comprenant un agent épaississant et présentant un caractère filant spécifique.

La présente invention a donc pour objet une composition cosmétique de revêtement des fibres kératiniques comprenant, dans un milieu aqueux physiologiquement acceptable, au moins un agent épaississant et des particules solides de cire ladite composition présentant un caractère filant dmax supérieur ou égal à 12 mm.

Le caractère filant représente l'aptitude de la composition selon l'invention à former, lors de l'application sur les fibres kératiniques, des fils qui, après étirement à l'aide d'une brosse, sont suffisamment consistants et conservent leur forme. En particulier, après application, ces fils ne se rétractent pas, ne se rompent pas, ni ne s'affaissent sous l'action de leur propre poids. Ces fils se forment dans le prolongement de chaque cil et permettent d'obtenir un effet allongeant remarquable.

L'invention se rapporte également à un procédé cosmétique de traitement ou de maquillage des fibres kératiniques comprenant l'application sur lesdites fibres kératiniques d'une composition cosmétique telle que décrite précédemment.

L'invention a également pour objet l'utilisation d'une composition cosmétique telle que décrite précédemment pour obtenir un film déposé sur les fibres kératiniques lisse et homogène et/ou un effet allongeant et/ou un effet séparant.

L'invention a encore pour objet l'utilisation, dans une composition cosmétique de revêtement des fibres kératiniques, d'un milieu aqueux physiologiquement acceptable et d'un agent épaississant, ladite composition présentant un caractère filant dmax supérieur ou égal à 12 mm, pour obtenir un film déposé sur les fibres kératiniques lisse et homogène et/ou un effet allongeant et/ou un effet séparant.

Par «milieu aqueux physiologiquement acceptable », on entend un milieu aqueux non toxique et compatible avec les fibres kératiniques, tels que les cils, les sourcils et les cheveux d'êtres humains, notamment compatible avec la zone oculaire.

<u>Mesure du caractère filant</u>

Le caractère filant de la composition est déterminé à température ambiante (25°C) à l'aide du texturomètre vendu sous la dénomination TA X- T2i par la société RHEO, équipé d'un mobile cylindrique en inox d'un diamètre de 1,2 cm, en imposant un déplacement vertical du mobile dans un échantillon de la composition (contenue dans un godet de 3,2 cm de diamètre rempli à son maximum, l'excès de composition étant arasé en surface) et en mesurant l'évolution de la force (force de compression ou force d'étirement) en fonction du temps.
Le protocole est le suivant :

Le mobile se déplaçe à une vitesse de 1 mm/s et pénétre dans la composition jusqu'à une profondeur de pénétration

de 0,2 mm. Le mobile est maintenu fixe pendant une seconde (correspondant à la phase de relaxation) puis est retiré à une vitesse de 10 mm/s.
Pendant la phase de relaxation, la force (force de compression) décroît fortement jusqu'à devenir nulle puis, lors du retrait du mobile (phase de retrait), la force (force d'étirement), devient négative pour ensuite croître à nouveau vers la valeur de 0.
Lors de la phase de retrait, un fil de composition se forme entre la surface de la composition et le mobile, la longueur du fil augmentant jusqu'à atteindre son maximum avant rupture. Le caractère filant ou dmax (exprimé en mm) correspond à la longueur maximale du fil avant rupture, équivalente à la distance parcourue par le mobile pendant cette phase de retrait.

$$d_{max}\ (mm) = \text{temps de retrait (s) x vitesse de retrait (mm/s)}$$

Le temps de retrait est le temps qui s'écoule à partir du retrait du mobile de l'échantillon (formation du fil de composition) jusqu'à la rupture du fil.
Les mesures du caractère filant sont répétées trois fois pour la même composition.
La composition selon l'invention présente un caractère filant dmax supérieur ou égal à 12 mm, allant notamment de 12 à 35 mm et mieux de 15 à 32 mm. La composition présentant un tel caractère filant selon l'invention permet l'obtention, lors de l'application sur les fibres kératiniques, en particulier les cils, à l'aide d'une brosse, de former un fil de composition dans le prolongement du cil. Après application sur le cil, ce fil conserve sa forme et ne se rétracte pas, ce qui permet l'obtention d'un effet d'allongement du cil.
La composition selon l'invention peut présenter un comportement rhéologique particulier, défini notamment par des caractéristiques viscoélastiques. Elle peut en outre présenter un profil d'écoulement spécifique, tel que décrit ci-après.

<u>Mesure des caractéristiques rhéologiques</u>

Les mesures ont été effectuées sur un rhéomètre à contrainte imposée, RS 75 de la société ThermoRhéo, équipé d'un bain thermostaté et d'un mobile en acier inoxydable à géométrie plan/plan, le plan ayant un diamètre de 20 mm et un entrefer (distance entre le plan inférieur - appelé plan stator - sur lequel est déposée la composition et le plan supérieur - appelé plan rotor) de 0,3 mm. Les 2 plans sont striés pour limiter les phénomènes de glissement aux parois des plans.
Les mesures sont effectuées à 25°C ± 0.5°C.

Mesure des caractéristiques viscoélastiques

Les compositions conformes à l'invention ont avantageusement un comportement viscoélastique.
De façon générale, un matériau est dit viscoélastique quand, sous l'effet du cisaillement, il possède à la fois les caractéristiques d'un matériau élastique, c'est à dire capable de stocker de l'énergie et les caractéristiques d'un matériau visqueux, c'est à dire capable de dissiper de l'énergie.
Le comportement viscoélastique des compositions conformes à l'invention peut être plus particulièrement caractérisé par son élasticité $\delta$ (exprimée en degré), qui correspondant à l'angle de déphasage entre la contrainte appliquée et la déformation enregistrée. Ce paramètre est notamment défini dans l'ouvrage "Initiation à la rhéologie", G. Couarraze et J.L. Grossiord, 2$^{ème}$ édition, 1991, Edition Lavoisier-Tec 1 Doc.
Le comportement viscoélastique des compositions selon l'invention est notamment caractérisé par une élasticité plateau $\delta p$ supérieure ou égale à 36°, par exemple allant de 36° à 75°, et mieux de 45 à 58°.
Les mesures dynamiques sont réalisées en appliquant une variation harmonique de la contrainte. Dans ces expériences, les amplitudes de la contrainte de cisaillement (notée $\tau$) et de la déformation de cisaillement (notée $\gamma$) sont faibles de manière à rester dans les limites du domaine viscoélastique linéaire de la composition (conditions permettant d'évaluer les caractéristiques rhéologiques de la composition au repos).
Le domaine linéaire viscoélastique est généralement défini par le fait que la réponse du matériau (i.e. la déformation) est à tout moment directement proportionnelle à la valeur de la force appliquée (i.e. la contrainte). Dans ce domaine, les contraintes appliquées sont faibles et le matériau subit des déformations sans modifier sa structure microscopique. Dans ces conditions, le matériau est étudié « au repos » et de façon non destructive.
La composition est soumise à un cisaillement harmonique selon une contrainte $\tau(t)$ variant de façon sinusoïdale selon une pulsation ? (? = 2? $\nu$, $\nu$ étant la fréquence du cisaillement appliqué). La composition ainsi cisaillée subie une contrainte $\tau(t)$ et répond selon une déformation ?(t) correspondant à des micro déformations pour lesquelles le module

de rigidité varie peu en fonction de la contrainte imposée.
La contrainte $\tau(t)$ et la déformation ?(t) sont définies respectivement par les relations suivantes :

$$\tau(t) = \tau_0 \cos(\omega \cdot t) \qquad \gamma(t) = \gamma_0 \cos(\omega \cdot t - \delta)$$

$\tau_0$ étant l'amplitude maximale de la contrainte et $\gamma_0$ étant l'amplitude maximale de la déformation. L'élasticité $\delta$ est l'angle de déphasage entre la contrainte et la déformation.
Les mesures sont effectuées à une fréquence de 1 Hz ($\nu$= 1 Hz).

On mesure ainsi l'évolution du module de rigidité G (correspondant au rapport de $\tau_0$ sur $\gamma_0$) et de l'élasticité $\delta$ (correspondant à l'angle de déphasage de la contrainte appliquée par rapport à la déformation mesurée) en fonction de la contrainte $\tau(t)$ appliquée.
On mesure en particulier la déformation de la composition pour la zone de contrainte dans laquelle la variation de l'élasticité $\delta$ est inférieure à 10% (zone des microdéformations) et on détermine ainsi le paramètre dit « plateau » $\delta_P$.

Mesure du profil d'écoulement

La composition selon l'invention présente avantageusement un profil rhéologique tel que, pour une gamme de gradient de cisaillement allant $10^{-2}$ à $10^3$ $s^{-1}$, les rapports de la variation du gradient de cisaillement sur la variation de la contrainte de cisaillement appliquée (noté $\frac{\Delta\dot{\gamma}}{\Delta\tau}$) sont au plus égaux à 7 ; ladite composition étant apte à subir des contraintes de cisaillement sans fracturation dans la gamme de gradient dé cisaillement.

L'analyse en régime d'écoulement à l'équilibre consiste à soumettre l'échantillon, à partir d'un instant donné, à une contrainte de cisaillement $\tau$ instantanée (cisaillement), maintenue constante pendant un temps t (temps d'attente choisi de façon à ce que le régime permanent soit atteint, t = 30s). Simultanément, on suit l'évolution au cours du temps de la déformation de cisaillement correspondante $\gamma$ et on enregistre le gradient de cisaillement $\dot{\gamma}$ lorsque l'équilibre est atteint.

Dans un premier temps, l'échantillon est mis en température à 25°C pendant 2 min (sans aucun cisaillement appliqué).
Puis l'on mesure l'écoulement à l'équilibre en mode contrainte imposée.
On applique des contraintes croissantes à l'échantillon en partant d'une contrainte initiale égale à 0.64 Pa pour arriver à une contrainte finale de 2000 Pa, les contraintes n'étant appliquées qu'une seule fois.
On attend l'obtention d'une valeur stable entre chaque contrainte, le temps d'attente entre chaque contrainte étant de 30 s.
La gamme de gradient de cisaillement va de $10^{-4}$ $s^{-1}$ à $10^3$ $s^{-1}$. Dans la gamme considérée, la valeur maximale de $10^3$ $s^{-1}$ doit être prise en compte avec une incertitude de mesure de $\pm$ 150 $s^{-1}$.

L'analyse des résultats se fait au travers de la représentation graphique de l'évolution de la viscosité, notée $\eta$, en fonction du gradient de cisaillement, noté $\dot{\gamma}$. Le graphe montre une première zone plateau, appelée première région newtonienne, définie pour les faibles valeurs de $\dot{\gamma}$($\dot{\gamma} \leq 10^{-3} s^{-1}$) où la viscosité reste constante : cette zone est représentative de la viscosité du produit au repos.
Pour les gradients de cisaillement plus élevés, $10^{-2} s^{-1} \leq \dot{\gamma} \leq 10^3\ s^{-1}$, la viscosité chute, le produit se met en mouvement pour s'écouler en se fluidifiant. , Dans cette fenêtre de cisaillement, deux comportements peuvent apparaître :
soit l'écoulement est homogène, au quel cas il y a équidistance entre les points du graphe et la courbe correspond à un rapport :

$$\frac{\Delta\dot{\gamma}}{\Delta\tau} \leq 7$$

soit l'écoulement est inhomogène et dans ce cas il n'y a plus continuité ni équidistance entre les ceints du graphe et la courbe correspond à un rapport :

$$\frac{\Delta\dot{\gamma}}{\Delta\tau} > 7$$

Avantageusement, la composition selon l'invention présente un profil rhéologique tel que, pour une gamme de gradient de cisaillement allant $10^{-2}$ à $10^3$ $s^{-1}$, les rapports de la variation du gradient de cisaillement sur la variation de la contrainte de cisaillement appliquée (noté $\frac{\Delta \dot{\gamma}}{\Delta \tau}$) sont au plus égaux à 7 ; ladite composition étant apte à subir des contraintes de cisaillement sans fracturation dans la gamme de gradient de cisaillement.

Ainsi, les compositions qui ont de telles caractéristiques, présentent un écoulement continu (c'est-à-dire sans fracturation) et homogène (c'est-à-dire sans formation de paquets). De telles compositions, appliquées sur les fibres kératiniques, conduisent à un dépôt lisse et homogène sur lesdites fibres,

Milieu aqueux physiologiquement acceptable

Le milieu aqueux physiologiquement acceptable des compositions selon l'invention est un milieu aqueux continu c'est-à-dire de l'eau ou un mélange d'eau avec au moins un solvant organique miscible à l'eau.
Le milieu aqueux de la composition peut ainsi comprendre un mélange d'eau et de solvant organique miscible à l'eau comme les monoalcools inférieurs ayant de 1 à 5 atomes de carbone tels que l'éthanol, l'isopropanol, les glycols ayant de 2 à 8 atomes de carbone tels que la glycérine, le propylène glycol, l'éthylène glycol, le 1,3-butylène glycol, le dipropylène glycol, les cétones en $C_3$-$C_4$, les aldéhydes en $C_2$-$C_4$. Le milieu aqueux (eau et
éventuellement le solvant organique miscible à l'eau) peut représenter, en pratique, de 10 % à 80 % en poids, par rapport au poids total de la composition, de préférence de 20 à 70% en poids, et mieux de 30 à 60% en poids.

Agent épaissant

L'agent épaississant de la composition selon l'invention est en particulier un agent épaississant rhéofluidifiant c'est-à-dire un agent apte à conférer à la composition le contenant un comportement rhéofluidifiant caractérisé par le fait que la viscosité de la composition diminue lorsque l'on applique à la composition des cisaillements croissants.

Avantageusement, l'agent épaississant est choisi parmi les polymères associatifs.

Par "polymère associatif' au sens de la présente invention, on entend tout polymère amphiphile comportant dans sa structure au moins une chaîne grasse et au moins une portion hydrophile.

Les polymères associatifs conformes à la présente invention peuvent être anioniques, cationiques, non-ioniques ou amphotères.

Parmi les polymères anioniques associatifs, on peut citer ceux comportant au moins un motif hydrophile, et au moins un motif éther d'allyle à chaîne grasse, plus particulièrement parmi ceux dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif éther d'allyle à chaîne grasse correspond au monomère de formule (I) suivante :

$$CH_2 = C(R')CH_2\ O\ B_n\ R \qquad (I)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement encore de 12 à 18 atomes de carbone
Des polymères amphiphiles anioniques de ce type sont décrits et préparés, selon un procédé de polymérisation en émulsion, dans le brevet EP-0 216 479.

Comme polymères anioniques associatifs, on peut citer également les polymères anioniques comportant au moins un motif hydrophile de type acide carboxylique insaturé oléfinique, et au moins un motif hydrophobe exclusivement de type ester d'alkyl ($C_{10}$-$C_{30}$) d'acide carboxylique insaturé.

On peut citer à titre d'exemple les polymères anioniques décrits et préparés, selon les brevets US-3 915 921 et 4 509 949.

Comme polymères associatifs cationiques, on peut citer les dérivés de cellulose quaternisée et les polyacrylates à groupements latéraux aminés.

Les polymères associatifs non-ioniques peuvent être choisis parmi :

- les celluloses modifiées par des groupements comportant au moins une chaîne grasse comme par exemple les hydroxyéthylcelluloses modifiées par des groupements comportant au moins une chaîne grasse tels que des groupes alkyle, notamment en $C_8$-$C_{22}$, arylalkyle, alkylaryle, telles que le NATROSOL PLUS GRADE 330 CS (alkyles en $C_{16}$) vendu par la société AQUALON,
- les celluloses modifiées par des groupes polyalkylène glycol éther d'alkyl phénol,

- les guars tels que l'hydroxypropyl guar, modifiés par des groupements comportant au moins une chaîne grasse telle qu'une chaîne alkyle,
- les copolymères de vinyl pyrrolidone et de monomères hydrophobes à chaîne grasse ;
- les copolymères de méthacrylates ou d'acrylates d'alkyles en $C_1$-$C_6$ et de monomères amphiphiles comportant au moins une chaîne grasse,
- les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle,
- les polyuréthanes associatifs
- leurs mélanges.

De préférence, le polymère associatif est choisi parmi les polyuréthanes associatifs.
Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
En particulier, ces polymères comportent au moins deux chaînes lipophiles hydrocarbonées, ayant de $C_6$ à $C_{30}$ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyuréthanes associatifs peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Ces polymères peuvent être également en greffons ou en étoile.
De préférence, les polyuréthanes associatifs sont des copolymères triblocs dont la séquence hydrophile est une chaîne polyoxyéthylénée comportant de 50 à 1 000 groupements oxyéthylénés. En général les polyuréthannes associatifs comportent une liaison uréthane entre les séquences hydrophiles, d'où l'origine du nom.
A titre d'exemple, des polymères associatifs utilisables dans l'invention, on peut citer le polymère $C_{16}$-$OE_{120}$-$C_{16}$ de la société SERVO DELDEN (sous le nom SER AD FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéclate 208 ou 204. Ces polyuréthannes associatifs sont vendus sous forme pure.
Le produit DW 1206B de chez RHOM & HAAS à chaîne alkyle en $C_{20}$ et à liaison uréthane, vendu à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le SER AD FX1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS.
Les polymères utilisables dans l'invention sont en particulier ceux décrits dans l'article de G. Fonnum, J. Bakke et Fk. Hansen - Colloid Polym. Sci 271, 380.389 (1993).
L'agent épaississant peut représenter de 0,05 à 20% en poids en matières sèches (ou matières actives) par rapport au poids total de la composition selon l'invention, de préférence de 0,2 à 10% en poids, et mieux de 0,5 à 5% en poids.

Particules solides

Avantageusement, la composition selon l'invention comprend une dispersion aqueuse de premières particules solides ayant une taille moyenne inférieure ou égale à 700 nm. Ces premières particules solides ont une taille moyenne inférieure à 700 nm, notamment allant de 10 à 700 nm, et mieux de 20 à 300 nm, la taille moyenne des particules étant exprimée en diamètre « effectif » moyen en volume D[4,3] tel que défini ci-après.
Les particules solides peuvent présenter des formes variées. Elles peuvent notamment être sphériques.
Ces particules sont dispersées dans le milieu aqueux physiologiquement acceptable de la composition.

Mesure des tailles de particules

Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer

un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La composition est caractérisée par son diamètre « effectif » moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25°C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre. Le diamètre « effectif » est obtenu en prenant un indice de réfraction de 1,33 pour l'eau et un indice de réfraction moyen de 1,42 pour les particules.

Avantageusement, la composition selon l'invention comprend en outre des secondes particules solides ayant une taille moyenne supérieure à 701 nm, en particulier de taille allant de 701 à 50 μm, de préférence de taille allant de 1 μm à 50 μm et mieux de 1 μm à 25 μm, ces secondes particules solides sont dispersées dans le milieu aqueux physiologiquement acceptable de la composition selon l'invention.

De préférence, les particules solides, c'est à dire les premières et secondes particules solides, sont choisies parmi les polymères filmogènes, les polymères semi-cristallins, les agents rhéologiques, les cires, les pigments, les charges et leurs mélanges.

Avantageusement, les premières particules sont choisies parmi les polymères filmogènes, les cires et leurs mélanges. Préférentiellement, les secondes particules sont choisies parmi les cires, les pigments, les charges, les polymères semi-cristallins, les agents rhéologiques, et leurs mélanges.

Avantageusement, les premières particules représentent de 5 à 30% en poids et mieux de 10 à 25 % en poids par rapport au poids total de la composition.

De préférence, les secondes particules représentent de 10 à 40% en poids et mieux de 12 à 30% en poids par rapport au poids total de la composition.

Cires

Les particules solides présentes dans la composition selon l'invention peuvent comprendre des particules de cires.

Par "cire" au sens de la présente invention, on entend un composé gras lipophile, solide à température ambiante (25°C), à changement d'état solide/liquide réversible, ayant un point de fusion supérieur ou égal à 30 °C pouvant aller jusqu'à 120°C et présentant à l'état solide une organisation cristalline anisotrope. En particulier, les cires convenant à l'invention peuvent présenter un point de fusion supérieur à 45 °C environ, et en particulier supérieur à 55°C.

Le point de fusion de la cire peut être mesuré à l'aide d'un calorimètre à balayage différentiel (D.S.C.), par exemple le calorimètre vendu sous la dénomination DSC 30 par la société METLER.

Le protocole de mesure est le suivant : un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides et rigides, à température ambiante d'origine animale, végétale, minérale ou de synthèse et leurs mélanges.

La cire peut également présenter une dureté allant de 0,05 MPa à 15 MPa, et de préférence allant de 6 MPa

à 15 MPa . La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.
Le protocole de mesure est le suivant : la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32 comme par exemple l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE. On peut encore citer les cires de silicone, les cires fluorées et leurs mélanges.

La composition selon l'invention contient généralement de 1 à 40 % en poids de cires, en particulier elle peut contenir de 5 à 30 %, et mieux de 5 à 20 % en poids par rapport au poids total de la composition.

Les particules de cires en dispersion aqueuse présentent notamment une taille allant de 10 nm à 50 μm.

Les premières particules solides peuvent être avantageusement choisies parmi des particules de cires, qui sont présentes sous la forme d'une microdispersion aqueuse de particules d'une ou plusieurs cires, et qui ont une taille moyenne inférieure ou égale à 700 nm, de préférence allant de 10 à 700 nm, mieux de 30 à 500 nm, et encore mieux de 50 à 200 nm.

La composition peut ainsi comprendre un mélange de premières particules d'une ou plusieurs cires de taille allant de 10 nm à 50 μm telles que décrite ci-dessus et de secondes particules d'une ou plusieurs cires ayant une taille moyenne allant de 1 μm à 50 μm, de préférence 1 μm à 25 μm.

<u>Polymères filmogènes</u>

Les particules solides présentes dans la composition selon l'invention peuvent comprendre des particules de polymère filmogène en dispersion aqueuse. Une telle dispersion est connue généralement sous le nom de latex ou pseudolatex.

La composition selon l'invention peut comprendre des particules d'un ou plusieurs polymère(s) filmogène(s).

Dans la présente demande, on entend par "polymère filmogène", un polymère apte à former à lui seul ou en présence d'un agent auxiliaire de filmification, un film continu et adhérent sur un support, notamment sur les fibres kératiniques comme les cils.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique et leurs mélanges. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .
Parmi les (méth)acrylates d'alkyle, on peut citer le méthacrylate de méthyle, le méthacrylate d'éthyle, le méthacrylate

de butyle, le méthacrylate d'isobutyle, le méthacrylate d'éthyl-2 hexyle, le méthacrylate de lauryle, le méthacrylate de cyclohexyle et leurs mélanges.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle et leurs mélanges.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique utilisable selon l'invention, on peut citer ceux vendus sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432®, DOW LATEX 424® par la société DOW CHEMICAL, DAITOSOL 5000 AD par la société DAITO KASEY KOGYO, "SYNTRAN® 5190 », « SYNTRAN® 5760 », « SYNTRAN®5009» par la société INTERPOLYMER.

Parmi les polycondensats filmogènes, on peut également citer les polyuréthanes, les polyesters, les polyesters amides, les polyesters à chaîne grasse, les polyamides, et les résines époxyesters. On utilise de préférence les polyuréthanes.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.
Le polyuréthane filmogène peut être, par exemple, un copolymère polyuréthane, polyurée-uréthane, ou polyurée, aliphatique, cycloaliphatique ou aromatique, comportant seule ou en mélange :

- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Les polyuréthanes filmogènes utilisables dans l'invention peuvent être également obtenus à partir de polyesters, ramifiés ou non, ou d'alkyles comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Comme polyuréthane filmogène utilisable selon l'invention, on peut utiliser ceux fabriqués ou commercialisés sous les dénominations NEOREZ R-981®, NEOREZ R-974® par la société AVECIA-NEORESINS, les AVALURE UR-405®, AVALURE UR-410®, AVALURE UR-425®, AVALURE UR-450®, SANCURE 875®, SANCURE 861®, SANCURE 878®, SANCURE 2060® par la société GOODRICH, IMPRANIL 85® par la société BAYER et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique et leurs mélanges. Ces monomères acide dicarboxylique peuvent être utilisés seuls ou en combinaison

d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanedio, et leurs mélanges. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, t'acide sulfotéréphtalique, l'acide sulfophtalique, l'acide 4-sulfonaphtalène-2,7-dicarboxylique et leurs mélanges.

On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique et leurs mélanges. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

De façon avantageuse, le polymère filmogène est choisi parmi les polyuréthanes filmogènes, les polyacryliques filmogènes décrits précédemment et leurs mélanges.

La dispersion aqueuse de particules d'un ou plusieurs polymères filmogènes peut être préparée par l'homme du métier sur base de ses connaissances générales.

Le polymère filmogène en dispersion aqueuse peut être présent dans la composition selon l'invention en une teneur en matières sèches (ou matières actives) de polymère allant de 0,1% à 30 % en poids par rapport au poids total de la composition, de préférence de 1% à 15% en poids, et mieux de 2% à 10% en poids.

La taille des particules de polymère filmogène en dispersion aqueuse peut aller de 10 nm à 700 nm, de préférence de 20 nm à 300 nm, et mieux de 25 à 150 nm.

La composition selon l'invention peut comprendre, en outre, un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

<u>Polymère semi-cristallin</u>

Les particules solides présentes dans la composition selon l'invention peuvent comprendre des particules (secondes particules) de polymères cristallin en dispersion aqueuse.

Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin selon l'invention a une température de fusion supérieure ou égale à 30˚C (notamment allant de 30˚C à 80˚C), de préférence allant de 30˚C à 60˚C. Cette température de fusion est une température de changement d'état du premier ordre. Cette température de fusion peut être mesurée par toute méthode connue et en

particulier à l'aide d'un calorimètre à balayage différentiel (D.S.C).

De façon avantageuse, le ou les polymères semi-cristallins auxquelles s'applique l'invention présentent une masse moléculaire moyenne en nombre supérieure ou égale à 1 000.

De façon avantageuse, le ou les polymères semi-cristailins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins.

Les polymères semi-cristallins utilisables dans l'invention sont en particulier :

- les copolymères séquencés de polyoléfines à cristallisation contrôlée, notamment ceux dont les monomères sont décrits dans EP-A-0 951 897.
- les polycondensats et notamment de type polyester, aliphatique ou aromatique ou copolyester aliphatique/aromatique,
- les homo- ou co-polymères portant au moins une chaîne latérale cristallisable et les homo-

ou co-polymères portant dans le squelette au moins une séquence cristallisable, comme ceux décrits dans le document US-A-5 156 911,

- les homo- ou co-polymères portant au moins une chaîne latérale cristallisable en particulier à groupement(s) fluoré (s) tels que décrits dans le document WO-A-01/19333, et leurs mélanges. Dans ces deux derniers cas, la ou les chaînes latérales ou séquences cristallisables sont hydrophobes.

*A) Polymères semi-cristallins à chaînes latérales cristallisables*

On peut citer en particulier ceux définis dans le document US-A-5156911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.

Ces homo- ou co-polymères sont de toute nature du moment qu'ils présentent les conditions indiquées précédemment. Ils peuvent résulter:

- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

  D'une façon générale, ces polymères sont choisis notamment parmi les homopolymères et copolymères résultant de la polymérisation d'au moins un monomère à chaîne(s) cristallisable(s) qui peut être représenté par la formule X :

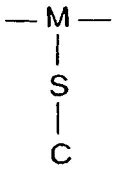

avec M représentant un atome du squelette polymérique, S représentant un espaceur, C représentant un groupe cristallisable.

Les chaînes « -S-C » cristallisables peuvent être aliphatiques ou aromatiques, éventuellement fluorées ou perfluorées. « S » représente notamment un groupe $(CH_2)_n$ ou $(CH_2CH_2O)_n$ ou $(CH_2O)$, linéaire ou ramifié ou cyclique, avec n entier allant de 0 à 22. De préférence « S » est un groupe linéaire. De préférence, « S » et « C » sont différents.

Lorsque les chaînes « -S-C » cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyles en $C_{14}$-$C_{24}$. Lorsqu'il s'agit de chaînes alkyle fluorées ou perfluorées, elles comportent au moins 6 atomes de carbone fluorés et notamment au moins 11 atomes de carbone dont au moins 6 atomes de carbone sont fluorés.

Comme exemple de polymères ou copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en $C_{14}$-$C_{24}$, les (méth)acrylates de perfluoroalkyle avec un groupe alkyle perfluoro en $C_{11}$-$C_{15}$, les N-alkyl (méth) acrylamides avec le groupe alkyle en $C_{14}$ à $C_{24}$ avec ou sans atome de fluor, les esters vinyliques à chaînes alkyle ou perfluoro (alkyle) avec le groupe alkyle en $C_{14}$ à $C_{24}$ (avec au moins 6 atomes de fluor pour une chaîne perfluoro alkyle), les éthers vinyliques à chaînes alkyle ou perfluoro (alkyle) avec le groupe alkyle en $C_{14}$ à $C_{24}$ et au moins 6 atomes de fluor pour une chaîne perfluoro alkyle, les alpha-oléfines en $C_{14}$ à $C_{24}$ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :

$\alpha$) de Y qui est un monomère polaire ou non polaire ou un mélange des deux :

- Lorsque Y est un monomère polaire, c'est soit un monomère porteur de groupes polyoxyalkylénés (notamment oxyéthyléné et/ou oxypropyléné), un (méth)acrylate d'hydroxyalkyte comme l'acrylate d'hydroxyéthyle, le (méth) acrylamide, un N-alkyl(méth)acrylamide, un N,N-dialkyl(méth)acrylamide comme par exemple le N,N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
- Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou styrène substitué par un groupe alkyle en $C_1$ à $C_{10}$, comme l'$\alpha$-méthylstyrène, un macromonomère du type polyorganosiloxane à insaturation vinylique.
  Par "alkyle», on entend au sens au sens de l'invention un groupement saturé notamment en $C_8$ à $C_{24}$, sauf mention exprès, et mieux en $C_{14}$ à $C_{24}$.

$\beta$) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d'alkyl(méth) acrylate ou d'alkyl(méth)acrylamide avec un groupe alkyle tel que défini ci-dessus, et notamment en $C_{14}$-$C_{24}$, des copolymères de ces monomères avec un monomère hydrophile de préférence de nature différente de l'acide (méth) acrylique comme la N-vinylpyrrolidone ou l'hydroxyéthyl (méth)acrylate et leurs mélanges.

*B) Les polymères portant dans le squelette au moins une séquence cristallisable* Ces polymères sont notamment des copolymères séquencés constitués d'au moins 2 séquences de nature chimique différente dont l'une est cristallisable.

- On peut utiliser les polymères séquencés définis dans le brevet US-A-5 156 911 ;
- Les copolymères séquencés d'oléfine ou de cyclooléfine à chaîne cristallisable comme ceux issus de la polymérisation séquencée de :

  cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
  avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
  et en particulier les copoly(éthylène/norbornène) blocs et les terpolymères (éthylènelpropylèneléthylidène-norbornène) blocs. On peut aussi utiliser ceux résultants de la copolymérisation séquencée d'au moins 2 $\alpha$-oléfines en $C_2$-$C_{16}$ et mieux en $C_2$-$C_{12}$ et encore mieux en $C_4$-$C_{12}$ tels que ceux cités précédemment et en particulier les bipolymères séquencés d'éthylène et d'1-octène.

- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :

  Séquence cristallisable par nature : a) polyester comme les poly(alkylène téréphtalate), b) polyoléfine comme les polyéthylènes ou polypropylènes.
  Séquence amorphe et lipophile comme les polyoléfines ou copoly(oléfine)s amorphes telles que le poly (isobutylène), le polybutadiène hydrogéné, le poly(isoprène) hydrogéné.

Comme exemple de tels copolymères à séquence cristallisable et à séquence amorphe distinctes, on peut citer :

$\alpha$) les copolymères séquencés poly(e-caprolactone)-b-poly(butadiène), utilisés de préférence hydrogénés, tels que ceux décrits dans l'article "Melting behavior of poly(e-caprolactone)-block-polybutadiène copolymers" de S. Nojima, Macromolécules, 32, 3727-3734 (1999).
$\beta$) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
$\gamma$) les copolymères séquencés poly(éthylène)-b-copoly(éthylène/propylène) cités dans les articles "Morphology of semi-crystalline block copolymers of ethylene-(ethylene-altpropylene)" de P. Rangarajan et al., Macromolecules, 26, 4640-4645 (1993) et " Polymer agregates with crystalline cores : the system poly(ethylene)-poly(ethylene-propylene)" de P. Richter et al., Macromolécules, 30, 1053-1068 (1997)..
$\delta$) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être ou non réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.
De préférence, les polymères semi-cristallins de la composition selon l'invention sont non réticulés.
Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en $C_{14}$ à $C_{24}$, les (méth)acrylates de perfluoroalkyle en $C_{11}$ à $C_{15}$, les N alkyl (méth)acrylamides en $C_{14}$ à $C_{24}$ avec ou sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alphaoléfines en $C_{14}$ à $C_{24}$, les para-alkyl styrènes avec un groupe alkyle comportant de 12 à 24 atomes de carbone, avec au moins un ester ou amide d'acide monocarboxylique en $C_1$ à $C_{10}$ éventuellement fluoré, qui peut être représenté par la formule suivante :

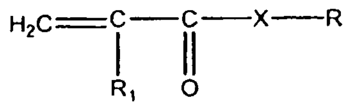

dans laquelle $R_1$ est H ou $CH_3$. R représente un groupe alkyle en $C_1$-$C_{10}$ éventuellement fluoré et X représente O, NH ou $NR_2$, où $R_2$ représente un groupe alkyle en $C_1$-$C_{10}$ éventuellement fluoré.

Selon un mode plus particulier de réalisation de l'invention, le polymère est issu d'un monomère à chaîne cristallisable choisi parmi les (méth)acrylates d'alkyle saturés en $C_{14}$ à $C_{22}$.

A titre d'exemple particulier de polymère semi-cristallin structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.

Les polymères semi-cristallins peuvent être notamment : ceux décrits dans les exemples 3, 4, 5, 7, 9, 13 du brevet US-A-5 156 911 à groupement -COOH, résultant de la copolymérisation d'acide acrylique et d'alkyl(méth)acrylate en $C_5$ à $C_{16}$ et plus particulièrement de la copolymérisation :

d'acide acrylique, d'hexadécylacrylate et d'isodécylacrylate dans un rapport pondéral 1/16/3,
. d'acide acrylique et de pentadécylacrylate dans un rapport pondéral 1/19,
. d'acide acrylique, d'hexadécylacrylate, éthylacrylate dans un rapport pondéral 2,5/76,5/20,
. d'acide acrylique, d'hexadécylacrylate et de méthylacrylate dans un rapport pondéral 5/85/10,
. d'acide acrylique et de octadécylméthacrylate dans un rapport pondéral 2,5/97,5 ,
. d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le structure « O » de National Starch tel que celui décrit dans le document US-A-5 736 125 de point de fusion 44°C ainsi que les polymères semi-cristallins à chaînes pendantes cristalisables comportant des groupements fluorés tels que décrits dans les exemples 1, 4, 6, 7 et 8 du document WO-A-01/19333.

On peut encore utiliser les polymères semi-cristallins de bas point de fusion obtenus par copolymérisation d'acrylate de stéaryle et d'acide acrylique ou de NVP tels que décrits dans le document US-A-5 519 063 ou EP-A-550745 et plus spécialement ceux décrits dans les exemples 1 et 2, ci-après, de préparation de polymère, de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5519063 et EP-A-550745 et plus spécialement ceux décrits dans les exemples 3 et 4, ci-après, de préparation de polymère, de température de fusion respectivement de 60°C et 58°C.

Le polymère semi-cristallin en dispersion aqueuse peut être présent dans la composition selon l'invention en une teneur en matières sèches (ou matières actives) de polymère allant de 1 à 40% en poids par rapport au poids total de la composition, de préférence de 5 à 30% en poids, et mieux de 5 à 20% en poids.

La taille des particules de polymère semi-cristallin en dispersion aqueuse peut aller de 1 μm à 50 μm, de préférence 1 μm à 25 μm.

Charges

Les particules solides présentes dans la composition selon l'invention peuvent comprendre des charges.

Par "charge", on entend toute particule incolore ou blanche choisie parmi les charges minérales ou organiques, lamellaires, sphériques ou oblongues, chimiquement inertes dans la composition.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® (ORGASOL de chez ATOCHEM), de poly-β-alanine et de polyéthylène, les poudres de polymères de tétrafluoroéthylène comme le Téflon®, la lauroyl-lysine, l'amidon, le nitrure de bore, les micro sphères creuses polymériques telles que celles de chlorure de polyvinylidène/acrylonitrile comme l'EXPANCEL® (NOBEL INDUSTRIE), les poudres acryliques telles que le POLYTRAP® (DOW CORNING), les particules de polyméthacrylate de méthyle et les microbilles de résine de silicone (TOSPEARLS® de TOSHIBA, par exemple), le carbonate de calcium précipité, le carbonate et l'hydro-carbonate de magnésium, l'hydroxyapatite, les microsphères de silice creuses (SILICA BF-ADS® de MAPRECOS), les microcapsules de verre ou de céramique, les savons métalliques dérivés d'acides organiques carboxyliques ayant de 8 à 22 atomes de carbone, de

préférence de 12 à 18 atomes de carbone, par exemple le stéarate de zinc, de magnésium ou de lithium, le laurate de zinc, le myristate de magnésium et leurs mélanges.

Les charges peuvent représenter de 0,1 à 25 %, et mieux de 1 à 20% en poids du poids total de la composition.

Les charges utilisées dans la composition selon l'invention ont de préférence une taille moyenne allant de 1 à 30 μm.

Pigments

Les particules solides présentes dans la composition selon l'invention peuvent être choisies parmi les pigments.

Par pigments, il faut comprendre des particules blanches ou colorées, minérales ou organiques, insolubles dans le milieu aqueux physiologiquement acceptable de la composition, destinées à colorer et/ou opacifier la composition. Les pigments comprennent aussi les nacres ou pigments nacrés qui sont des particules irisées, notamment produites par certains mollusques dans leur coquille ou bien synthétisées.

On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome, le bleu ferrique et leurs mélanges. Parmi les pigments organiques, on peut citer le noir de carbone, les pigments de type D & C, et les laques à base de carmin de cochenille, de baryum, strontium, calcium, aluminium et leurs mélanges. On peut également utiliser des pigments traités avec un agent hydrophobe. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges. Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine Le terme alkyl mentionné dans les composés cités précédement désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

On peut aussi utiliser les pigments à effet optique particulier comme les particules de verre recouvertes de métal, notamment d'or, d'argent et de platine.

Les nacres ou pigments nacrés sont des particules irisées, notamment produites par certains mollusques dans leur coquille ou bien synthétisées, insolubles dans le milieu physiologiquement acceptable de la composition. Ils peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth et leurs mélanges. On peut également utiliser les pigments interférentiels, notamment à cristaux liquides ou multicouches.

Les pigments peuvent représenter de 0,01 à 25 %, de préférence de 0,1 à 20 % et mieux de 1 à 10 % en poids du poids total de la composition.

Agent rhéologique

Les secondes particules peuvent en outre comprendre un agent rhéologique.

Cet agent rhéologique est avantageusement choisi parmi les gélifiants lipophiles.

Le gélifiant lipophile peut être organique ou minéral, polymérique ou moléculaire.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium.

Les gélifiants lipophiles organiques polymériques sont par exemple les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; l'éthylcellulose comme celles vendues sous le nom d'Ethocel par Dow Chemical ; les polyamides tels que les copolymères d'un diacide en $C_{36}$ condensé sur l'éthylène diamine de masse moléculaire moyenne en poids d'environ 6000 tels que les composés commercialisés par la société Arizona Chemical sous les noms Uniclear 80 et Uniclear 100, les gommes notamment siliconées comme les PDMS ayant une viscosité > 100 000 centistokes, les galactommananes comportant de un à six

et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et leurs mélanges.

Comme gélifiant lipophile préféré, on utilise des gélifiants organique moléculaires non polymériques, également appelés organogélateurs, associés à une phase grasse liquide, qui sont des composés dont les molécules sont capables d'établir entre elles des interactions physiques conduisant à une auto-agrégation des molécules avec formation d'un réseau supra-moléculaire 3D qui est responsable de la gélification de la phase grasse liquide.

Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides température ambiante, appelés aussi huiles, généralement compatibles entre eux.

La ou les huiles peu(ven)t être choisie(s) parmi toutes les huiles physiologiquement acceptables et en particulier cosmétiquement acceptables, notamment les huiles minérales, animales, végétales, synthétiques ; en particulier les huiles hydrocarbonées et/ou siliconées et/ou fluorées volatiles ou non volatiles et leurs mélanges. Plus précisément, par « huile hydrocarbonée », on entend une huile comportant principalement des atomes de carbone et d'hydrogène et éventuellement une ou plusieurs fonctions choisies parmi les fonctions hydroxyle, ester, éther, carboxylique. Généralement, l'huile présente une viscosité de 0,5 à 100 000 cps, de préférence de 50 à 50 000 cps et de préférence encore de 100 à 300 000 cps.

A titre d'exemple d'huile utilisable dans l'invention, on peut citer :

- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam ;
- les esters et les éthers de synthèse notamment d'acides gras comme les huiles de formule $R_1COOR_2$ dans laquelle $R_1$ représente le reste d'un acide gras supérieur comportant de 1 à 40 atomes de carbone et $R_2$ représente une chaîne hydrocarbonée contenant de 1 à 40 atomes de carbone avec $R_1 + R_2 \geq 10$ comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates,
- leurs mélanges.

Le réseau supra-moléculaire peut résulter de la formation d'un réseau de fibrilles (dues aux empilements ou agrégations de molécules d'organogélateur), immobilisant les molécules de la phase grasse liquide.

L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.

Les interactions physiques sont diverses mais excluent la co-cristallisation. Ces interactions physiques sont en particulier des interactions du type interactions hydrogènes auto-complémentaires, interactions n entre cycles insaturés, interactions dipolaires, liaisons de coordination avec des dérivés organométalliques et leurs associations. En général, chaque molécule d'un organogélateur peut établir plusieurs types d'interactions physiques avec une molécule voisine. Aussi, avantageusement, les molécules des organogélateurs selon l'invention comportent au moins un groupement capable d'établir des liaisons hydrogènes et mieux au moins deux groupements capables d'établir des liaisons hydrogène, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire

des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans la composition selon l'invention sont notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02147031.

On peut notamment citer parmi ces organogélateurs, les amides d'acides carboxyliques en particulier les acides tri-carboxyliques comme les cyclohexanetricarboxamides (voir la demande de brevet européen EP-A-1068854), les diamides ayant des chaînes hydrocarbonées contenant chacune de 1 à 22 atomes de carbone, par exemple de 6 à 18 atomes de carbone, lesdites chaînes étant non substituées ou substituées avec au moins un substituant choisi parmi les groupes ester, urée et fluoro (voir la demande EP-A-1086945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides résultant de l'action d'un N-acylamino acide avec des amines comportant de 1 à 22 atomes de carbone, comme par exemple ceux décrits dans le document WO-93123008 et notamment les amides de l'acide N-acylglutamique où le groupe acyle représente une chaîne alkyle en $C_8$ à $C_{22}$ tels que le dibutylamide de l'acide N-Lauroyl-L-glutamique, fabriqué ou commercialisé par la société Ajinomoto sous la dénomination GP-1 et leurs mélanges.

Les compositions peuvent contenir de 0,05 à 10% d'agent rhéologique, de préférence, de 0,1 à 5% en poids, et mieux de 0,5 à 3% en poids par rapport au poids total de la composition.

Autres ingrédients

La composition selon l'invention peut en outre comprendre des fibres qui permettent une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport UD (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, de préférence de 0,1 mm à 5 mm et mieux de 1 mm à 3,5 mm. Leur section peut être comprise dans un cercle de diamètre allant de 2 nm à 500 μm, de préférence allant de 100 nm à 100 μm et mieux de 1 μm à 50 μm. Le poids ou titre des fibres est souvent donné en denier ou décitex et représente le poids en gramme pour 9 km de fil. De préférence, les fibres selon l'invention ont un titre choisi dans la gamme allant de 0,15 à 30 deniers et mieux de 0,18 à 18 deniers.

Les fibres peuvent être celles utilisées dans la fabrication des textiles et notamment des fibres de soie, de coton, de laine, de lin, des fibres de cellulose - notamment extraites notamment du bois, des légumes ou des algues -, de rayonne, de polyamide (Nylon®), de viscose, d'acétate notamment d'acétate de rayonne, de poly-(p-phénylène-téréphtalamide) (ou d'aramide) notamment de Kevlar®, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et caprolactone ("Monocryl" de chez Johnson & Johnson) ; les fibres synthétiques résorbables du type copolymère d'acide lactique et d'acide glycolique ("Vicryl" de chez Johnson & Johnson) ; les fibres de polyester téréphtalique ("Ethibond" de chez Johnson & Johnson) et les fils d'acier inoxydable ("Acier" de chez Johnson & Johnson) notamment pour une application en vernis à ongles.

Par ailleurs, les fibres peuvent être traités ou non en surface, enrobées ou non. Comme fibres enrobées utilisables dans l'invention, on peut citer des fibres de polyamides enrobées de sulfure de cuivre pour un effet anti-statique (par exemple le "R-STAT" de chez Rhodia) ou un autre polymère permettant une organisation particulière des fibres (traitement de surface spécifique) ou traitement de surface induisant des effets de couleurs/hologrammes (fibre "Lurex" de chez Sildorex, par exemple).

De préférence, on utilise des fibres d'origine synthétiques et en particulier des fibres organiques, comme celles utilisées en chirurgie. Avantageusement, on peut utiliser des fibres insolubles dans l'eau.

Les fibres utilisables dans la composition selon l'invention sont préférentiellement des fibres de polyamide, de cellulose, de poly-p-phénylène téréphtalamide ou de de polyéthylène. Leur longueur (L) peut aller de 0,1 mm à 5 mm, de préférence de 0,25 mm à 1,6 mm et leur diamètre moyen peut aller de 1 μm à 50 μm. En particulier, on peut utiliser les fibres de polyamide commercialisées par les Etablissements P. Bonte sous le nom de "Polyamide 0.9 Dtex 3 mm", ayant un diamètre moyen de 6 μm, un titre d'environ 0.9 dtex et une longueur allant de 0,3 mm à 5 mm. On peut aussi utiliser les fibres de celluloses (ou de rayonne) ayant un diamètre moyen de 50 μm et une longueur allant de 0,5 mm à 6 mm comme celles vendues sous le nom de "Natural rayon flock fiber RC1 BE - N003 - M04" par la société Claremont Flock. On peut également utiliser des fibres de polyéthylène comme celles vendues sous le nom de "Shurt Stuff 13 099 F" par la société Mini Fibers.

La composition selon l'invention peut également comprendre des fibres dites "rigides", par opposition aux fibres citées précédemment, qui ne sont pas des fibres rigides. Les fibres rigides, initialement sensiblement droites, lorsqu'elles sont placées dans un milieu dispersant, ne voient pas leur forme sensiblement modifiée, ce qui se traduit par la condition angulaire définie ci-après, reflétant une forme que l'on peut qualifier de toujours, sensiblement droite, linéaire. Cette condition d'angle reflète la rigidité des fibres qui peut difficilement être exprimée par un autre paramètre pour des objets ayant une taille aussi faible que les fibres rigides.

La rigidité des fibres se traduit par la condition angulaire suivante : avantageusement, au moins 50 % en nombre, de préférence au moins 75 % en nombre, et mieux au moins 90 % en nombre des fibres sont telles que l'angle formé entre la tangente à l'axe central longitudinal de la fibre et la droite reliant ladite extrémité au point sur l'axe central longitudinal de la fibre correspondant à la moitié de la longueur de la fibre soit inférieur à 15 ° et l'angle formé entre la tangente à l'axe central longitudinal de la fibre en un point situé à mi-longueur de la fibre et la droite reliant l'une des extrémités au point sur l'axe central longitudinal de la fibre correspondant à la moitié de la longueur de fibre soit inférieur ou égal à 15 °, pour une même longueur de fibre allant de 0,8 mm à 5 mm, de préférence allant de 1 mm à 4 mm, de préférence allant de 1 mm à 3 mm, et mieux de 2 mm.

Avantageusement, l'angle, mentionné ci-dessus, est mesuré aux deux extrémités de la fibre et en un point situé à mi-longueur de la fibre, en d'autres termes, on effectue dans ce cas trois mesures et la moyenne des angles mesurés est inférieure ou égale à 15°.

Notamment, la tangente, en tout point de la fibre, forme un angle inférieur à 15°.

Dans la présente demande, l'angle formé par la tangente en un point de la fibre est l'angle formé entre la tangente à l'axe central longitudinal de la fibre audit point de la fibre et la droite reliant l'extrémité de la fibre la plus proche dudit point au point sur l'axe central longitudinal de la fibre correspondant à la moitié de la longueur de la fibre.

Généralement, les fibres rigides utilisables dans la composition selon l'invention ont la même longueur de fibre ou une longueur sensiblement identique.

Plus précisément lorsque l'on observe au microscope, avec un objectif permettant un grossissement de 2,5 et avec une vision plein champ, un milieu dans lequel sont dispersées les fibres rigides à une concentration des fibres de 1 % en poids, un nombre majoritaire de fibres rigides, c'est-à-dire au moins 50 % en nombre des fibres rigides, de préférence au moins 75 % en nombre des fibres rigides, et mieux au moins 90 % en nombre des fibres rigides, doivent satisfaire à la condition angulaire définie plus haut. La mesure conduisant à la valeur de l'angle est effectuée pour une même longueur de fibres, cette longueur est comprise dans la gamme allant de 0,8 mm à 5 mm, de préférence de 1 à 4 mm, de préférence de 1 à 3 mm, et mieux de 2 mm.

Le milieu dans lequel est réalisée l'observation est un milieu dispersant assurant une bonne dispersion des fibres rigides, par exemple de l'eau, un gel aqueux d'argile ou de polyuréthane associatif. On peut même réaliser une observation directe de la composition contenant les fibres rigides. Un échantillon de la composition ou de la dispersion préparée est placée entre lame et lamelle pour l'observation au microscope avec un objectif permettant un grossissement de 2,5 et avec une vision plein champ. La vision plein champ permet de voir les fibres dans leur intégralité.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques.

Comme exemples de fibres rigides, on peut citer les fibres :

- de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;
- de polyamide, telles que celles vendues sous les dénominations TRILOBAL NYLON 0.120-1.8 DPF ; TRILOBAL NYLON 0.120-18 DPF ; NYLON 0.120-6 DPF par la société Cellusuede products ; ou obtenues par découpe de fils vendus sous la dénomination FIBRE NOMEX BRAND 430 TAILLE 3 MM par la société DUPONT DE NEMOURS ;
- de polyimide-amide telles que celles vendues sous les dénomination "KERMEL", KERMEL TECH" par la société

RHODIA ;
- de poly-(p-phénylène-téréphtalamide) (ou d'aramide) notamment vendues sous la dénomination Kevlar® par la société DUPONT DE NEMOURS ;
- des fibres à structure multicouche comprenant des couches alternées de polymères choisis parmi les polyesters, les polymères acryliques, les polyamides, tellles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Les fibres rigides particulièrement préféres sont les fibres de polymide-amide aromatiques.
Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auquels on pourra se référer.
Les fibres de polyimide-amide aromatique préférées sont des fibres de polyimide-amide comprenant des motifs répétitif de formule :

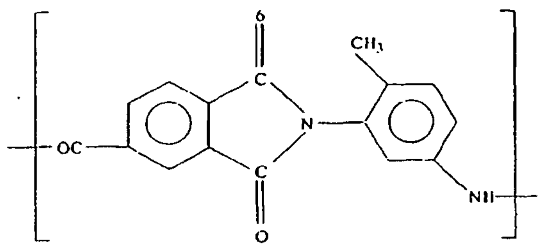

obtenu par polycondensation du toluylène diisocyanate et de l'anhydride triméllitique.
Les fibres peuvent êtres présentes dans la composition selon l'invention en une teneur allant de 0,01 % à 10 % en poids, par rapport au poids total de la composition, de préférence de 0,1 % à 5 % en poids, et mieux de 0,3 % à 3 % en poids.
La composition selon l'invention peut en outre comprendre d'autres ingrédients habituellement utilisés en cosmétique. De tels ingrédients peuvent être notamment des agents plastifiants, des agents de coalescence, des colorants liposolubles ou hydrosolubles, des tensio-actifs, des conservateurs, des huiles, des agents hydratants, des parfums, et leurs mélanges, qui sont bien connus de l'état de la technique.
Bien entendu, l'homme du métier veillera à choisir ce ou ces ingrédients et/ou leur quantité, de manière telle que les propriétés avantageuses de la composition selon l'invention ne soient pas, ou substantiellement pas, altérées par l'adjonction envisagée.
Les compositions selon l'invention peuvent aussi contenir un ou plusieurs adjuvants usuels tels que des agents alcalinisants ou acidifiants, des agents de textures, des additifs d'étalement, des plastifiants ainsi que des ingrédients actifs hydrosolubles utilisés habituellement dans les préparations cosmétiques pour les fibres kératiniques.
La composition selon l'invention peut être préparée selon les méthodes usuelles des domaines considérés.
Les exemples qui suivent illustrent de manière non limitative la présente invention.
Les quantités indiquées sont en pourcentage pondéral et exprimées par rapport au poids total de la composition, sauf instructions contraires.
Les mesures de rhéologie ont été effectuées sur un rhéomètre à contrainte imposée Haake RS 75 dans les conditions suivantes :

température de mesure : 25 °C
balayage en contrainte de 1 à 2000 Pa
fréquence de mesure : 1 Hz.

**<u>Exemple 1 :</u>** Préparation d'une microdispersion de cire d'abeille

On prépare une microdispersion de cires d'abeille ayant la composition suivante

| | |
|---|---|
| Cire d'abeille | 40% |

Suite de tableau

| | | |
|---|---|---|
| Stéarate de sodium | | 4% |
| Conservateur | | 0.4% |
| Eau | qsp | 100 |

**Mode opératoire**

La cire, le stéarate de sodium et le conservateur sont chauffés à une température de 75-80°C et grossièrement dispersés pendant 2 min dans une cuve sous agitation (pales et turbine) à 1500 tr/min. L'ensemble est ensuite passé plusieurs fois à l'homogénéisateur haute pression, en maintenant une température de 75-80°C, jusqu'à stabilisation de la pression (pression 1$^{er}$ étage : 700 bar, 2$^{e}$ étage : 320 bar).
On refroidit à température ambiante (refroidisseur à plaque).

Les particules de cire de cette microdispersion présentent une taille de 134 nm.

**Exemple 2 : Composition de mascara**

On a préparé un mascara ayant la composition suivante :

| | |
|---|---|
| Copolymère alcool stéarylique oxyéthyléné (100 OE)/polyéthylène glycol (136 OE) hexaméthylène diisocyanate ** (SER AD FX 1100 de la société SERVO DELDEN) | 2% |
| Dispersion aqueuse de polyuréthane aliphatique à 38% en matières actives (AVALURE UR-450 de la société NOVEON-GOODRICH), taille des particules : environ 66 nm ) | 8% (m. a)* |
| Micro-dispersion de cire de l'exemple 1 | 10% (m.a) |
| Cire d'abeille | 10% |
| Oxyde de fer noir | 5% |
| Propylène glycol | 5% |
| Poudre de polytetrafluoroéthylène (Téflon ®) | 5% |
| Ethanol | 5% |
| Conservateurs (propylparaben/methylparaben) | qs |
| Eau | qsp 100 |

*m.a = matière active

**polymère associatif

Taille des particules de cire d'abeille : environ 5-10 μm,
Taille des particules de charge (poudre de polytetrafluoroéthylène) : environ 8-13 μm.

Mode opératoire

Le polymère associatif (SER AD FX 1100) est dispersé dans l'eau à 40°C de façon à obtenir un gel puis on laisse revenir le mélange à température ambiante sous agitation.
On ajoute ensuite, toutjours sous agitation, le latex (AVALURE UR-450) puis l'oxyde de fer noir qui a été préalablement dispersé dans le propylène glycol et passé au broyeur tricylindre.
On laisse le mélange sous agitation 15 min (on vérifie l'état de dispersion du pigment au microscope) puis on monte la température à 70°C.

Parallèlement, on fait fondre la cire et le conservateur à une température d'environ 100°C, puis cette phase cireuse est ajoutée au mélange On laisse l'ensemble sous agitation pendant 15 min, puis on refroidit progressivement jusqu'à la température ambiante.

On ajoute ensuite la microdispersion de cire de l'exemple 1, le conservateur dilué dans l'alcool puis la poudre de polytetrafluoroéthylène.

Ce mascara présente un caractère filant de 16,7 mm et une élasticité δ égale à 36° et présente un écoulement continu et homogène.

Ce mascara a été jugé comme présentant une texture fluide et brillante et permet, après application sur les cils, d'obtenir un film lisse et homogène ainsi qu'un allongement satisfaisant des cils.

**Exemple 3 : Composition de mascara**

On a préparé un mascara ayant la composition suivante :

| | |
|---|---|
| Copolymère alcool stéarylique oxyéthyléné (100 OE)/ polyéthylène glycol (136 OE) hexaméthylène diisocyanate (SER AD FX 1100 de la société SERVO DELDEN) | 2% |
| Dispersion aqueuse de copolymères acryliques et styrène/acrylique à 40% en matières actives (SYNTRAN 5760 de la société INTERPOLYMER), taille des particules : environ 48 nm | 12% (m. a)* |
| Micro-dispersion de cire de l'exemple 1 | 10% (m.a) |
| Cire d'abeille | 6% |
| Oxyde de fer noir | 5% |
| Propylène glycol | 5% |
| Ethanol | 5% |
| Conservateurs (propylparabenlmethylparaben) | qs |
| Eau | qsp 100 |
| *m.a = matière active | |

On utilise le même mode opératoire que dans l'exemple précédent.

Ce mascara présente un caractère filant de 12 mm et une élasticité δ égale à 42˚, il présente un écoulement continu et homogène.

Une fois appliqué sur les cils, cette composition de mascara forme un film homogène, sans paquets, et permet une bonne séparation et un allongement "cil à cil".

**Exemple 4 : Composition de mascara**

On a préparé un mascara ayant la composition suivante :

| | |
|---|---|
| Copolymère alcool stéarylique oxyéthyléné (100 OE)/ polyéthylène glycol (136 OE) hexaméthylène diisocyanate (SER AD FX 1100 de la société SERVO DELDEN) | 1,5% |
| Dispersion aqueuse de polyuréthane aliphatique à 38% en matières actives (AVALURE UR-450 de la société NOVEON-GOODRICH), taille des particules : environ 66 nm | 6% (m.a)* |
| Micro-dispersion de cire de l'exemple 1 | 10% (m.a) |
| Cire d'abeille | 11% |
| Oxyde de fer noir | 5% |
| Propylène glycol | 5% |
| Copolymère éthylène diamine/dimère de dilinoléate de stéaryle | 0,75% |
| (UNICLEAR 100 VG de la société la société ARIZONA CHEMICAL) Poudre de polytetrafluoroéthylène (Téflon ®) | 7% |
| Ethanol + Conservateurs (propylparaben/methylparaben) | qs |
| Eau | qsp 100 |

Mode opératoire

Le polymère associatif (SER AD FX 1100) est dispersé dans l'eau à 40˚C de façon à obtenir un gel puis on laisse revenir le mélange à température ambiante sous agitation.
On ajoute ensuite, toujours sous agitation, le latex (AVALURE UR-450) puis l'oxyde de fer noir qui a été préalablement dispersé dans le propylène glycol et passé au broyeur tricylindre.
On laisse le mélange sous agitation 15 min ( on vérifie l'état de dispersion du pigment au microscope) puis on monte la température à 70˚C.
Parallèlement, on fait fondre la cire, l'Uniclear 100 VG et le conservateur à une température d'environ 100˚C, puis cette phase cireuse est ajoutée au mélange On laisse l'ensemble sous agitation pendant 15 min, puis on refroidit progressivement jusqu'à la température ambiante.
On ajoute ensuite la microdispersion de cire de l'exemple 1, le conservateur dilué dans l'alcool puis la poudre de polytetrafluoroéthylène.

Ce mascara présente un caractère filant de 30 mm, une élasticité δ égale à 54° et un écoulement continu et homogène comme le montre le graphe ci-dessous.

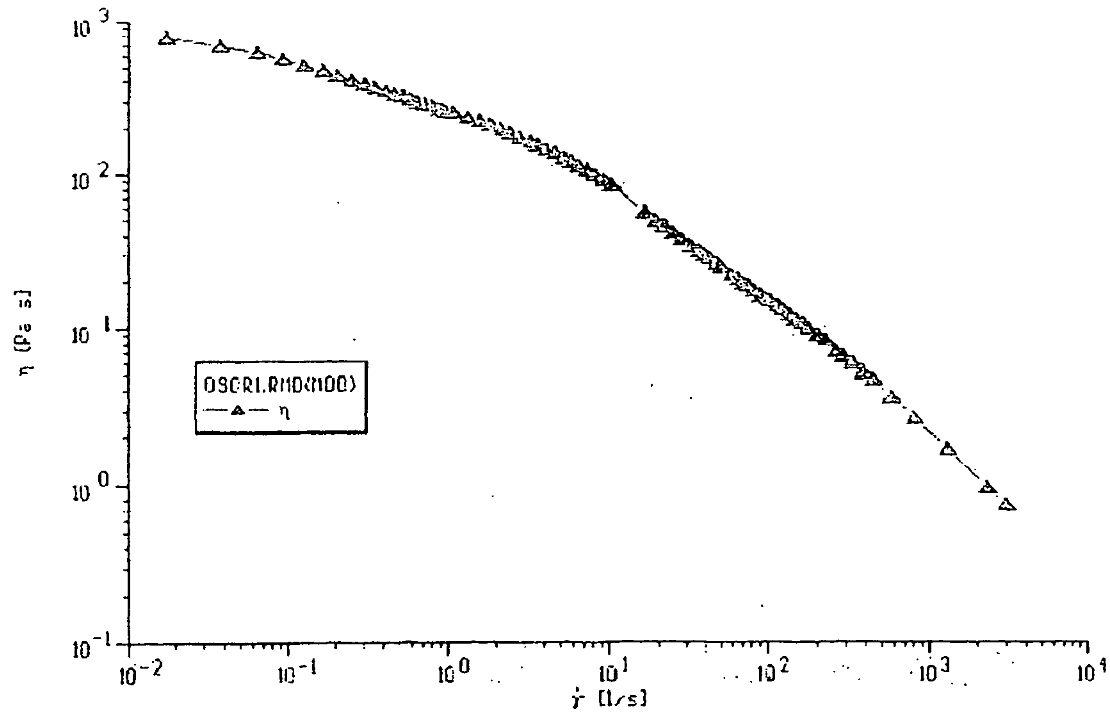


Pour cet exemple, $\frac{\Delta\dot{\gamma}}{\Delta\tau}$ varie de 7,9 $10^{-3}$ à 6,88 dans la gamme des cisaillements compris entre $1.10^{-2}s^{-1}$ et $1.10^{3}s^{-1}$

Le mascara de cet exemple est capable de supporter des cisaillements élevés de l'ordre de $1000s^{-1}$.
La figure 1 illustre le profil d'écoulement de ladite composition, le graphe représentant la viscosité (η en Pa.s) de la composition en fonction du gradient de cisaillement $\dot{\gamma}$ (en $s^{-1}$).
Elle confirme que l'écoulement est continu et homogène, les points étant équidistants sur la courbe.

Ce mascara a été jugé comme conférant, après application sur les cils, un film de composition de lisse et homogène ainsi qu'un allongement et une séparation des cils tout a fait satisfaisants.

## Revendications

1. Composition cosmétique de revêtement des fibres kératiniques comprenant, dans un milieu aqueux physiologiquement acceptable, au moins un agent épaississant et des particules solides de cire, ladite composition présentant un caractère filant dmax supérieur ou égal à 12 mm.

2. Composition selon la revendication précédente, **caractérisée en ce que** le caractère filant dmax va de 12 à 35 mm.

3. Composition selon l'une des revendications précédentes, **caractérisée en ce que** l'agent épaississant est choisi parmi les polymères associatifs.

4. Composition selon la revendication précédente, **caractérisée en ce que** le polymère associatif est choisi parmi les polymères associatifs non ioniques.

5. Composition selon la revendication 3 ou 4, **caractérisée en ce que** le polymère associatif est choisi parmi les polyuréthanes associatifs.

6. Composition selon la revendication précédente, **caractérisée en ce que** le polyuréthane associatif est un copolymère tribloc ayant une chaîne polyoxyéthylénée comportant de 50 à 1 000 groupements oxyéthylénés.

7. Composition selon l'une des revendications précédentes, **caractérisée en ce que** l'agent épaississant représente de 0,05 à 20% en poids en matières sèches par rapport au poids total de la composition, de préférence de 0,2 à 10% en poids, et mieux de 0,5 à 5% en poids.

8. Composition selon l'une des revendications précédentes, **caractérisée en ce qu'**elle comprend une dispersion aqueuse de premières particules solides ayant une taille moyenne inférieure ou égale à 700 nm.

9. Composition selon la revendication 8 **caractérisée en ce que** les premières particules sont choisies parmi les cires, les polymères filmogènes et leurs mélanges.

10. Composition selon la revendication 9, **caractérisée en ce que** le polymère filmogène représente de 0,1% à 30 % en poids par rapport au poids total de la composition, de préférence de 1% à 15% en poids, et mieux de 2% à 10% en poids.

11. Composition selon l'une des revendications précédentes **caractérisée en ce qu'**elle comprend en outre des secondes particules solides ayant une taille moyenne supérieure ou égale à 701 nm.

12. Composition selon la revendication 11 **caractérisée en ce que** les secondes particules sont choisies parmi les cires, les pigments, les charges, les polymères semi-cristallins, et leurs mélanges.

13. Composition selon la revendication 12, **caractérisée en ce que** la charge est choisie parmi le talc, le mica, la silice, le kaolin, les poudres de polyamide, de poly-β-alanine et de polyéthylène, les poudres de polymères de tétrafluoroéthylène, la lauroyl-lysine, l'amidon, le nitrure de bore, les micro sphères creuses polymériques, les poudres acryliques, les particules de polyméthacrylate de méthyle et les microbilles de résine de silicone, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium et leurs mélanges.

14. Composition selon la revendication 12 ou 13, **caractérisée en ce que** la charge représente de 0,1 à 25 %, et mieux de 1 à 20% en poids du poids total de la composition.

15. Composition selon la revendication 12, **caractérisée en ce que** le polymère semi-cristallin est choisi parmi :

   - les copolymères séquencés de polyoléfines à cristallisation contrôlée,
   - les polycondensats de type polyester, aliphatique ou aromatique ou copolyester aliphatique/aromatique,
   - les homo- ou co-polymères portant au moins une chaîne latérale cristallisable et les homo- ou co-polymères portant dans le squelette au moins une séquence cristallisable,
   - les homo- ou co-polymères portant au moins une chaîne latérale cristallisable en particulier à groupement(s) fluoré(s),

   et leurs mélanges.

16. Composition selon la revendications 15, **caractérisée en ce que** le polymère semi-cristallin est présente en une teneur en matières sèches de polymère allant de 1 à 40% en poids par rapport au poids total de la composition, de préférence de 5 à 30% en poids, et mieux de 5 à 20% en poids

17. Composition selon la revendication 12, **caractérisée en ce que** les pigments représentent de 0,01 à 25 %, de préférence de 0,1 à 20 % et mieux de 1 à 10% en poids du poids total de la composition.

18. Composition selon l'une revendications 11 ou 12, **caractérisée en ce que** les secondes particules comprennent en outre un agent rhéologique choisi parmi les gélifiants lipophiles.

19. Composition selon la revendication 18, **caractérisée en ce que** le gélifiant lipophile est choisi parmi les copolymères d'un diacide en $C_{36}$ condensé sur l'éthylène diamine de masse moléculaire moyenne en poids d'environ 6000.

**20.** Composition selon la revendication 18 ou 19, **caractérisée en ce** l'agent rhéologique représente de 0,05 à 10%, de préférence de 0,1 à 5% en poids, et mieux de 0,5 à 3% en poids par rapport au poids total de la composition.

**21.** Composition selon la revendication 9 ou 12, **caractérisée en ce que** la cire est choisie parmi la cire d'abeilles, la cire de lanoline, et les cires d'insectes de Chine; la cire de riz, la cire de Camauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines, l'ozokérite; les cires de polyéthylène, les cires obtenues par la synthèse de Fisher-Tropsch, les copolymères cireux ainsi que leurs esters, les cires obtenues par hydrogénation catalytique d'huiles animales ou végétales ayant des chaînes grasses, linéaires ou ramifiées, en C8-C32, les cires de silicone, les cires fluorées et leurs mélanges.

**22.** Composition selon la revendications 21, **caractérisée en ce que** la cire représente de 1 à 40 % en poids, en particulier de 5 à 30 %, et mieux de 5 à 20% en poids par rapport au poids total de la composition.

**23.** Composition selon la revendication 8, **caractérisée en ce que** les premières particules représentent de 5 à 30% en poids et mieux de 10 à 25 % en poids par rapport au poids total de la composition.

**24.** Composition selon la revendication 11, **caractérisée en ce que** les secondes particules représentent de 10 à 40% en poids et mieux de 12 à 30% en poids par rapport au poids total de la composition.

**25.** Composition selon l'une des revendications précédentes, **caractérisé en ce qu'**elle présente une élasticité plateau δp supérieure ou égale à 36°.

**26.** Composition selon la revendication précédente, **caractérisé en ce qu'**elle présente une élasticité plateau δp allant de 36° à 75°.

**27.** Composition selon l'une des revendications précédentes, **caractérisé en ce qu'**elle présente un profil rhéologique tel que, pour une gamme de gradient de cisaillement allant $10^{-2}$ à $10^3$ $s^{-1}$, les rapports de la variation du gradient de cisaillement sur la variation de la contrainte de cisaillement appliquée (noté $\frac{\Delta\dot{\gamma}}{\Delta\tau}$) sont au plus égaux à 7 ; ladite composition étant apte à subir des contraintes de cisaillement sans fracturation dans toute la largeur de ladite gamme de gradient de cisaillement.

**28.** Procédé cosmétique de traitement ou de maquillage des fibres kératiniques comprenant l'application sur lesdites fibres kératiniques d'une composition cosmétique selon l'une des revendications 1 à 27.

**29.** Utilisation d'une composition cosmétique selon l'une des revendications 1 à 27 pour obtenir un film déposé sur les fibres kératiniques lisse et homogène et/ou un effet allongeant et/ou un effet séparant.

**30.** Utilisation, dans une composition cosmétique de revêtement des fibres kératiniques, d'un milieu aqueux physiologiquement acceptable et d'un agent épaississant, ladite composition présentant un caractère filant dmax supérieur ou égal à 12 mm, pour obtenir un film déposé sur les fibres kératiniques lisse et homogène et/ou un effet allongeant et/ou un effet séparant.

**31.** Procédé cosmétique de maquillage des cils consistant à former sur les cils un film homogène d'une composition cosmétique comprenant, dans un milieu aqueux physiologiquement acceptable, au moins un agent épaississant, ladite composition présentant un caractère filant dmax supérieur ou égal à 12 mm.

**Patentansprüche**

**1.** Kosmetische Zusammensetzung zum Überziehen von Keratinfasern, die in einem physiologisch akzeptablen, wässrigen Medium mindestens ein Verdickungsmittel und feste Wachspartikel enthält, wobei die Zusammensetzung einen Faden ziehenden Charakter dmax von 12 mm oder darüber aufweist.

**2.** Zusammensetzung nach dem vorhergehenden Anspruch, **dadurch gekennzeichnet, dass** der Faden ziehende Charakter dmax im Bereich von 12 bis 35 mm liegt.

3. Zusammensetzung nach einem der vorhergehenden Ansprüche, **dadurch gekennzeichnet, dass** das Verdickungsmittel unter den assoziativen Polymeren ausgewählt ist.

4. Zusammensetzung nach dem vorhergehenden Anspruch, **dadurch gekennzeichnet, dass** das assoziative Polymer unter den nichtionischen assoziativen Polymeren ausgewählt ist.

5. Zusammensetzung nach Anspruch 3 oder 4, **dadurch gekennzeichnet, dass** das assoziative Polymer unter den assoziativen Polyurethanen ausgewählt ist.

6. Zusammensetzung nach dem vorhergehenden Anspruch, **dadurch gekennzeichnet, dass** das assoziative Polyurethan ein Dreiblock-Copolymer ist, das eine Polyoxyethylenkette aufweist, die 50 bis 100 Ethylenoxidgruppen aufweist.

7. Zusammensetzung nach einem der vorhergehenden Ansprüche, **dadurch gekennzeichnet, dass** das Verdickungsmittel 0,05 bis 20 Gew.-% Trockensubstanz, bezogen auf das Gesamtgewicht der Zusammensetzung, vorzugsweise 0,2 bis 10 Gew.-% und besser 0,5 bis 5 Gew.-% aufweist.

8. Zusammensetzung nach einem der vorhergehenden Ansprüche, **dadurch gekennzeichnet, dass** sie eine wässrige Dispersion von ersten festen Partikeln mit einer mittleren Größe von höchstens 700 nm enthält.

9. Zusammensetzung nach Anspruch 8, **dadurch gekennzeichnet, dass** die ersten Partikel unter den Wachsen, filmbildenden Polymeren und deren Gemischen ausgewählt sind.

10. Zusammensetzung nach Anspruch 9, **dadurch gekennzeichnet, dass** das filmbildende Polymer 0,1 bis 30 Gew.-%, bezogen auf das Gesamtgewicht der Zusammensetzung, vorzugsweise 1 bis 15 Gew.-% und besser 2 bis 10 Gew.-% ausmacht.

11. Zusammensetzung nach einem der vorhergehenden Ansprüche, **dadurch gekennzeichnet, dass** sie ferner zweite feste Partikel mit einer mittleren Größe von mindestens 701 nm aufweist.

12. Zusammensetzung nach Anspruch 11, **dadurch gekennzeichnet, dass** die zweiten Partikel unter den Wachsen, Pigmenten, Füllstoffen, halbkristallinen Polymeren und deren Gemischen ausgewählt sind.

13. Zusammensetzung nach Anspruch 12, **dadurch gekennzeichnet, dass** der Füllstoff unter Talk, Glimmer, Kieselsäure, Kaolin, Polyamidpulver, Poly-β-alaninpulver und Polyethylenpulver, den Pulvern von Tetrafluorethylenpolymeren, Lauroyllysin, Stärke, Bornitrid, polymeren Mikrohohlkugeln, Acrylpulvern, Polymethylacrylatpartikeln und Siliconharzmikrokugeln, gefälltem Calciumcarbonat, Magnesiumcarbonat, Magnesiumhydrogencarbonat, Hydroxyapatit, Siliciumoxidmikrohohlkugeln, Glasmikrokapseln, Keramikmikrokapseln, Metallseifen, die von organischen Carbonsäuren mit 8 bis 22 Kohlenstoffatomen und vorzugsweise 12 bis 18 Kohlenstoffatomen abgeleitet sind, beispielsweise Zinkstearat, Magnesiumstearat oder Lithiumstearat, Zinklaurat, Magnesiummyristat, und deren Gemischen ausgewählt ist.

14. Zusammensetzung nach Anspruch 12 oder 13, **dadurch gekennzeichnet, dass** der Füllstoff 0,1 bis 25 % und besser 1 bis 20 Gew.-% des Gesamtgewichts der Zusammensetzung ausmacht.

15. Zusammensetzung nach Anspruch 12, **dadurch gekennzeichnet, dass** das halbkristalline Polymer ausgewählt ist unter:

- sequentiellen Copolymeren von Polyolefinen mit kontrollierter Kristallisation,
- Polykondensaten vom Typ der aliphatischen oder aromatischen Polyester oder aliphatischen/aromatischen Copolyester,
- Homo- oder Copolymeren, die mindestens eine kristallisierbare Seitenkette aufweisen, und Homo- oder Copolymeren, die in dem Grundgerüst mindestens eine kristallisierbare Sequenz aufweisen,
- Homo- oder Copolymeren, die mindestens eine kristallisierbare Seitenkette insbesondere mit einer oder mehreren fluorierten Gruppen aufweisen,

und deren Gemischen.

**16.** Zusammensetzung nach Anspruch 15, **dadurch gekennzeichnet, dass** das halbkristalline Polymer in einem Trokkensubstanzgehalt des Polymers von 1 bis 40 Gew.-%, bezogen auf das Gesamtgewicht der Zusammensetzung, vorzugsweise 5 bis 30 Gew.-% und besser 5 bis 20 Gew.-% vorliegt.

**17.** Zusammensetzung nach Anspruch 12, **dadurch gekennzeichnet, dass** die Pigmente 0,01 bis 25 %, vorzugsweise 0,1 bis 20 % und besser 1 bis 10 Gew.-% des Gesamtgewichts der Zusammensetzung ausmachen.

**18.** Zusammensetzung nach einem der Ansprüche 11 oder 12, **dadurch gekennzeichnet, dass** die zweiten Partikel ferner ein Rheologiemittel umfassen, das unter den lipophilen Gelbildnern ausgewählt ist.

**19.** Zusammensetzung nach Anspruch 18, **dadurch gekennzeichnet, dass** der lipophile Gelbildner unter den Copolymeren einer $C_{36}$-Disäure, die mit Ethylendiamin kondensiert wurde, mit einer gewichtsmittleren Molmasse von etwa 6 000 ausgewählt ist.

**20.** Zusammensetzung nach Anspruch 18 oder 29, **dadurch gekennzeichnet, dass** das Rheologiemittel 0,05 bis 10 %, vorzugsweise 0,1 bis 5 Gew.-% und besser 0,5 bis 3 Gew.-%, bezogen auf das Gesamtgewicht der Zusammensetzung, ausmacht.

**21.** Zusammensetzung nach Anspruch 9 oder 12, **dadurch gekennzeichnet, dass** das Wachs unter Bienenwachs, Lanolinwachs, Chinawachsen, Reiswachs, Carnaubawachs, Canelillawachs, Ouricuriwachs, Alfawachs, Korkfaserwachs, Zuckerrohrwachs, Japanwachs und Sumachwachs; Montanwachs, mikrokristallinen Wachsen, Paraffinen, Ozokerit; Polyethylenwachsen, durch Fischer-Tropsch-Synthese hergestellten Wachsen, wachsartigen Copolymeren sowie deren Estern, Wachsen, die durch katalytische Hydrierung von tierischen oder pflanzlichen Ölen hergestellt werden, die geradkettig oder verzweigte $C_{8-32}$-Fettketten aufweisen, Siliconwachsen, fluorierten Wachsen und deren Gemischen, ausgewählt ist.

**22.** Zusammensetzung nach Anspruch 21, **dadurch gekennzeichnet, dass** das Wachs 1 bis 40 Gew.-%, insbesondere 5 bis 30 % und besser 5 bis 20 Gew.-%, bezogen auf das Gesamtgewicht der Zusammensetzung, ausmacht.

**23.** Zusammensetzung nach Anspruch 8, **dadurch gekennzeichnet, dass** die ersten Partikel 5 bis 30 Gew.-% und besser 10 bis 25 Gew.-%, bezogen auf das Gesamtgewicht der Zusammensetzung, ausmachen.

**24.** Zusammensetzung nach Anspruch 11, **dadurch gekennzeichnet, dass** die zweiten Partikel 10 bis 40 Gew.-% und besser 12 bis 30 Gew.-%, bezogen auf das Gesamtgewicht der Zusammensetzung, ausmachen.

**25.** Zusammensetzung nach einem der vorhergehenden Ansprüche, **dadurch gekennzeichnet, dass** sie eine Elastizität Plateau $\delta p$ von mindestens 36° besitzen.

**26.** Zusammensetzung nach dem vorhergehenden Anspruch, **dadurch gekennzeichnet, dass** sie eine Elastizität Plateau $\delta p$ von 36° bis 75° aufweisen.

**27.** Zusammensetzung nach einem der vorhergehenden Ansprüche, **dadurch gekennzeichnet, dass** sie ein rheologisches Profil besitzt, das so ist, dass für einen Bereich des Schergradienten von $10^{-2}$ bis $10^3$ $s^{-1}$ die, Verhältnisse der Änderung des Schergradienten zur Veränderung der angewandten Scherkraft (als $\frac{\Delta\dot{\gamma}}{\Delta\tau}$ bezeichnet) mindestens 7 betragen, wobei die Zusammensetzung, in dem gesamten Bereich des Schergradienten ohne Fraktionierung Scherbeanspruchungen ausgesetzt werden kann.

**28.** Kosmetisches Verfahren zur Behandlung oder zum Schminken von Keratinfasern, das das Aufbringen einer kosmetischen Zusammensetzung nach einem der Ansprüche 1 bis 27 auf die Fasern umfasst.

**29.** Verwendung einer kosmetischen Zusammensetzung nach einem der Ansprüche 1 bis 27 zur Bildung eines auf den Keratinfasern abgeschiedenen Films, der glatt und homogen ist und/oder verlängernd wirkt und/oder eine Trennwirkung besitzt.

**30.** Verwendung eines physiologisch akzeptablen wässrigen Mediums und eines Verdickungsmittels in einer kosmetischen Zusammensetzung zum Überziehen von Keratinfasern, wobei die Zusammensetzung einen Faden ziehenden Charakter dmax von mindestens 12 mm aufweist, um einen auf den Keratinfasern abgeschiedenen Film zu bilden, der glatt und homogen ist und/oder verlängernd wirkt und/oder eine Trennwirkung besitzt.

**31.** Kosmetisches Verfahren zum Schminken der Wimpern, das darin besteht, auf den Wimpern einen homogenen Film einer kosmetischen Zusammensetzung zu bilden, die in einem physiologisch akzeptablen wässrigen Medium mindestens ein verdickungsmittel enthält, wobei die Zusammensetzung einen filmbildenden Charakter dmax von mindestens 12 mm besitzt.

## Claims

**1.** Cosmetic composition for coating keratin fibres, comprising, in a physiologically acceptable aqueous medium, at least one thickener and solid particles of wax, the said composition having a threading nature dmax of greater than or equal to 12 mm.

**2.** Composition according to the preceding claim, **characterized in that** the threading nature dmax ranges from 12 to 35 mm.

**3.** Composition according to either of the preceding claims, **characterized in that** the thickener is chosen from associative polymers.

**4.** Composition according to the preceding claim, **characterized in that** the associative polymer is chosen from nonionic associative polymers.

**5.** Composition according to Claim 3 or 4, **characterized in that** the associative polymer is chosen from associative polyurethanes.

**6.** Composition according to the preceding claim, **characterized in that** the associative polyurethane is a triblock copolymer with a polyoxyethylenated chain comprising from 50 to 1000 oxyethylene groups.

**7.** Composition according to one of the preceding claims, **characterized in that** the thickener represents from 0.05% to 20% by weight of solids, preferably from 0.2% to 10% by weight and better still from 0.5% to 5% by weight of solids relative to the total weight of the composition.

**8.** Composition according to one of the preceding claims, **characterized in that** it comprises an aqueous dispersion of first solid particles with a mean size of less than or equal to 700 nm.

**9.** Composition according to Claim 8, **characterized in that** the first particles are chosen from waxes, film-forming polymers, and mixtures thereof.

**10.** Composition according to Claim 9, **characterized in that** the film-forming polymer represents from 0.1% to 30% by weight, preferably from 1% to 15% by weight and better still from 2% to 10% by weight, relative to the total weight of the composition.

**11.** Composition according to one of the preceding claims, **characterized in that** it also comprises second solid particles with a mean size of greater than or equal to 701 nm.

**12.** Composition according to Claim 11, **characterized in that** the second particles are chosen from waxes, pigments, fillers and semi-crystalline polymers, and mixtures thereof.

**13.** Composition according to Claim 12, **characterized in that** the filler is chosen from talc, mica, silica, kaolin, polyamide powder, poly-β-alanine powder and polyethylene powder, tetrafluoroethylene polymer powders, lauroyllysine, starch, boron nitride, hollow polymer microspheres, acrylic powders, polymethyl methacrylate particles and silicone resin microbeads, precipitated calcium carbonate, magnesium carbonate, magnesium hydrocarbonate, hydroxyapatite, hollow silica microspheres, glass or ceramic microcapsules, metal soaps derived from carboxylic organic acids containing from 8 to 22 carbon atoms and preferably from 12 to 18 carbon atoms, for example zinc stearate, magnesium stearate, lithium stearate, zinc laurate or magnesium myristate, and mixtures thereof.

**14.** Composition according to Claim 12 or 13, **characterized in that** the filler represents from 0.1% to 25% and better, still from 1% to 20% by weight of the total weight of the composition.

**15.** Composition according to Claim 12, **characterized in that** the semi-crystalline polymer is chosen from:

- block copolymers of polyolefins with controlled crystallization,
- polycondensates of aliphatic or aromatic polyester or of aliphatic/aromatic copolyester type,
- homopolymers or copolymers bearing at least one crystallizable side chain and homopolymers or copolymers bearing at least one crystallizable block in the skeleton,
- homopolymers or copolymers bearing at least one crystallizable side chain, in particular containing fluoro group(s),

and mixtures thereof.

**16.** Composition according to Claim 15, **characterized in that** the semi-crystalline polymer is present in a polymer solids content ranging from 1% to 40% by weight, preferably from 5% to 30% by weight and better still from 5% to 20% by weight, relative to the total weight of the composition.

**17.** Composition according to Claim 12, **characterized in that** the pigments represent from 0.01% to 25%, preferably from 0.1% to 20% and better still from 1% to 10% by weight of the total weight of the composition.

**18.** Composition according to either of Claims 11 and 12, **characterized in that** the second particles also comprise a rheological agent chosen from lipophilic gelling agents.

**19.** Composition according to Claim 18, **characterized in that** the lipophilic gelling agent is chosen from copolymers of a $C_{36}$ diacid condensed with ethylenediamine, with a weight-average molecular mass of about 6000.

**20.** Composition according to Claim 18 or 19, **characterized in that** the rheological agent represents from 0.05% to 10%, preferably from 0.1% to 5% by weight and better still from 0.5% to 3% by weight, relative to the total weight of the composition.

**21.** Composition according to Claim 9 or 12, **characterized in that** the wax is chosen from beeswax, Lanolin wax, Chinese insect waxes; rice wax, carnauba wax, candelilla wax, ouricury wax, esparto grass wax, cork fibre wax, sugar cane wax, Japan wax and sumach wax; montan wax, microcrystalline waxes, paraffins or ozokerite; polyethylene waxes, the waxes obtained by Fisher-Tropsch synthesis, waxy copolymers and esters thereof, the waxes obtained by catalytic hydrogenation of animal or plant oils containing linear or branched $C_8$-$C_{32}$ fatty chains, silicone waxes and fluoro waxes, and mixtures thereof.

**22.** Composition according to Claim 21, **characterized in that** the wax represents from 1% to 40% by weight, in particular from 5% to 30% by weight and better still from 5% to 20% by weight relative to the total weight of the composition.

**23.** Composition according to Claim 8, **characterized in that** the first particles represent from 5% to 30% by weight and better still from 10% to 25% by weight relative to the total weight of the composition.

**24.** Composition according to Claim 11, **characterized in that** the second particles represent from 10% to 40% by weight and better still from 12% to 30% by weight relative to the total weight of the composition.

**25.** Composition according to one of the preceding claims, **characterized in that** it has a plateau elasticity δp of greater than or equal to 36°.

**26.** Composition according to the preceding claim, **characterized in that** it has a plateau elasticity δp ranging from 36° to 75°.

**27.** Composition according to one of the preceding claims, **characterized in that** it has a rheological profile such that, for a shear rate range ranging from $10^{-2}$ to $10^{3}$ $s^{-1}$, the ratios of the variation in the shear rate to the variation in the

applied shear stress (noted as $\frac{\Delta\dot{\gamma}}{\Delta\tau}$) are not more than 7; the said composition being capable of undergoing shear stresses without fracturing throughout the said shear rate range.

**28.** Cosmetic treatment or makeup process for keratin fibres, comprising the application to the said keratin fibres of a cosmetic composition according to one of Claims 1 to 27.

**29.** Use of a cosmetic composition according to one of Claims 1 to 27 to obtain a smooth and uniform film deposited on keratin fibres and/or a lengthening effect and/or a separating effect.

**30.** Use, in a cosmetic composition for coating keratin fibres, of a physiologically acceptable aqueous medium and of a thickener, the said composition having a threading nature dmax of greater than or equal to 12 mm, to obtain a smooth and uniform film deposited on the keratin fibres and/or a lengthening effect and/or a separating effect.

**31.** Cosmetic makeup process for eyelashes, consisting in forming, on the eyelashes, a uniform film of a cosmetic composition comprising, in a physiologically acceptable medium, at least one thickener, the said composition having a threading nature dmax of greater than or equal to 12 mm.